(19) Europäisches Patentamt
European Patent Office
Office européen des brevets

(11) EP 2 407 460 B1

(12) **EUROPEAN PATENT SPECIFICATION**

(45) Date of publication and mention of the grant of the patent:
**17.07.2013 Bulletin 2013/29**

(51) Int Cl.:
***C07D 251/52*** (2006.01) ***C07K 1/10*** (2006.01)

(21) Application number: **10195135.8**

(22) Date of filing: **15.12.2010**

(54) **N-TRIAZINYLAMMONIUM SALTS, A METHOD OF PREPARATION AND USES THEREOF**

N-TRIAZINYLAMMONIUMSALZE, VERFAHREN ZUR IHRER HERSTELLUNG UND IHRE VERWENDUNG

SELS DU N-TRIAZINYLAMMONIUM, PROCÉDÉ DE PRÉPARATION, ET LEURS UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.07.2010 PL 39183210**

(43) Date of publication of application:
**18.01.2012 Bulletin 2012/03**

(73) Proprietor: **Politechnika Lódzka**
**90-924 Lódz (PL)**

(72) Inventors:
- **Kaminski, Zbigniew J.**
  **94-050, Lodz (PL)**
- **Zajac, Krzysztof**
  **44-200 Rybnik (PL)**
- **Paneth, Piotr**
  **92-713 Lodz (PL)**

(74) Representative: **Kiciak, Krzysztof Boleslaw**
**Przedsiebiorstwo Rzecznikow Patentowych Patpol sp. z o.o.**
**ul. Nowoursynowska 162 J**
**PL-02-776 Warszawa (PL)**

(56) References cited:
**EP-A1- 1 586 566**
**WO-A1-01/96282**
**WO-A1-2007/051496**
**CH-A- 423 708**
**GB-A- 1 013 231**
**GB-A- 1 104 134**
**JP-A- 60 181 374**
**JP-A- 60 181 377**
**US-A- 3 178 254**

- **KUNISHIMA M ET AL: "Approach to green chemistry of DMT-MM: recovery and recycle of coproduct to chloromethane-free DMT-MM", TETRAHEDRON LETTERS, ELSEVIER, AMSTERDAM, NL, vol. 43, no. 18, 29 April 2002 (2002-04-29), pages 3323-3326, XP004349376, ISSN: 0040-4039, DOI: DOI:10.1016/S0040-4039 (02)00546-4**
- **KUNISHIMA M ET AL: "4-(4,6-Dimethoxy-1,3,5-triazin-2-yl)-4-me thyl-morpholinium Chloride: An Efficient Condensing Agent Leading to the Formation of Amides and Esters", TETRAHEDRON, ELSEVIER SCIENCE PUBLISHERS, AMSTERDAM, NL, vol. 55, no. 46, 12 November 1999 (1999-11-12), pages 13159-13170, XP004180916, ISSN: 0040-4020, DOI: DOI:10.1016/S0040-4020(99)00809-1**

EP 2 407 460 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

The invention relates to N-triazinylammonium salts, a method of preparation and uses thereof.

Well-known triazinylammonium salts are widely employed as condensation reagents useful in the synthesis of amides, esters, carboxylic acid anhydrides, amino aldehydes, amino alcohols, peptides, nucleic acids and as reagents for protecting functional groups.

The N-triazinylammonium salts of hydrochloric, tetrafluoroboric, hexafluorophosphoric, and perchloric acid are known, *inter alia,* from the publications in Biopolymers, 55 (2) 140-165, and Australian Journal of Chemical Society, 54, 469 (2001) as well as from numerous patent descriptions.

Quaternary N-triazinylammonium salts of sulphonic acids are known from Polish patent application No. P 366673 and from European patent No. EP 1586566. These salts are manufactured in the reaction of sulphonic acid salts with quaternary triazinylammonium chlorides or by simultaneous treating of sulphonic acid salts with 2-chloro-4,6-disubstituted-1,3,5-triazines and tertiary amines.

A process for manufacturing of triazinylammonium salts consisting in treating of 2-chloro-4,6-dimethoxy-1,3,5-triazine and 2-chloro-4,6-diphenoxy-1,3,5-triazine with tertiary amines was described in Journal of the Organic Chemistry, 63, 4248-4255 (1998).

A process for manufacturing of triazinylammonium salts *via* reaction of 2-chloro-4,6-dimethoxy-1,3,5-triazine with trimethylamine is known from Chemistry of Heterocyclic Compounds, 38, 177-182 (2002).

In the same journal, namely Chemistry of Heterocyclic Compounds, 13, 802-805 (1977), manufacturing of triazinylammonium salts by reacting trimethylamine with the chloro derivatives of 2,4-dialkylamino-1,3,5-triazines, 2-alkylamino-4-methoxy-1,3,5-triazines and 2-alkylamino-4-alkylthio-1,3,5-triazines was disclosed.

A synthesis of 4-(4,6-dimethoxy-1,3,5-triazin-2-yl)-4-methylmorpholinium chloride consisting in the reaction of 2-chloro-4,6-dimethoxy-1,3,5-triazine with N-methylmorpholine, is known from Tetrahedron, 55, 13159-13170 (1999).

Tetrahedron Letters, 43, 3323-3326 (2002) discloses a process of N-triazinylammonium salts regeneration from the condensation by-product, namely from 2-hydroxy-4,6-dimethoxy-1,3,5-triazine.

A process for manufacturing of triazinylammonium salts, consisting in the treatment of 2-chloro-4,6-dimethoxy-1,3,5-triazine with piperazine is known from publication WO 2001096282 A1 and from *Chemical Abstracts,* **136** 37408 (2002).

According to patent specification No. US 6,458,948 B1 and Japanese patent specification No. JP 34634/1972, triazinylammonium perchlorates and tetrafluoroborates are manufactured by the treatment of appropriate triazinylammonium chlorides with sodium perchlorate or sodium tetrafluoroborate.

A publication in the Journal of the American Chemical Society, 127 (48); 16912-16920 (2005) discloses a process for manufacturing of N-triazinylammonium tetrafluoroborates in solution and in solid phase, as well as uses thereof in the synthesis of peptides.

The N-triazinylammonium salts, which are described in the above-mentioned publications, consist of two fragments, i.e., a positively charged N-triazinylammonium fragment and an anionic fragment such as chloride, perchlorate, tetrafluoroborate, trifluoromethanesulphonic, toluenesulphonic anions and others. It is well known that the structure of both fragments, *i.e.*, the cationic fragment and the anionic one, determines the reactivity and other properties of N-triazinylammonium salts. Since both fragments are joined by an ionic bond, it is possible to replace any of these fragments by employing for that purpose the procedures described in the chemical literature. Usually, as substrates for such replacement N-triazinylammonium chlorides are used, which are readily available. They are obtainable in the reaction of 2-chloro-4,6-disubstituted 1,3,5-triazines with the corresponding tertiary amines. The process of exchanging the chloride anion for other anions may lead to obtaining mixtures of salts. It is difficult to use these mixtures as reagents in stoichiometric amounts. Moreover, using them as reagents may lead, under unfavourable conditions, to regeneration of N-triazinylammonium chlorides or other salts susceptible to degradation processes. In order to minimise this risk, relatively expensive silver salts are used that in the presence of chloride anions form sparingly soluble precipitates of silver chloride. Alternatively, either lithium salts are used that form lithium chloride, which is more soluble in organic solvents in comparison to the poorly soluble N-triazinylammonium salts, or a multiphase ion exchange is used, as described in patent application No. PCT/EP2005/055793.

N-Triazinylammonium salts are used as vigorous condensation reagents in the synthesis of esters, amides, carboxylic anhydrides and peptides, both in solution and in solid phase. Examples of use of N-triazinylammonium salts are described in all of the above-mentioned publications. According to information published in Journal für Praktische Chemie 332, 579-581, (1990), the condensations mediated by N-triazinylammonium salts proceed *via* 2-acyloxy-4,6-substituted triazines, which are superactive esters.

In many applications quaternary N-triazinylammonium salts of sulphonic acids, known from patent No. EP 1586566, are good condensation reagents. A possibility of exchanging of the sulphonic acid-derived anion for another anion is a disadvantage, as it was discussed above at the description of the method of their preparation. The ion-exchange process proceeds vigorously and essentially without activation energy, thus leading to a mixture of products having an unpredictable reactivity. In an unfavourable case, the sulphonic acid-derived anion can be replaced with an anion of high nucleophilicity,

promoting dealkylation of an N-triazinylammonium salt and formation of by-products devoid of activity as condensation reagents.

Patent specifications GB 1 013 231, JP 60 181377, JP 60 181374, GB 1 104 134, US 3 178 254 and CH 423 708 disclose triazine derivatives for use as reactive dyes.

The invention relates to inner quaternary N-triazinylammonium salts of sulphonic acids, having a betaine structure, as represented by the Formula 1

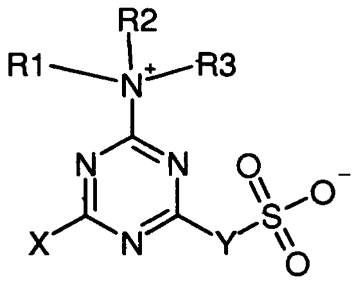

(Formula 1)

wherein

$R_1$, $R_2$ and $R_3$ are the substituents of a tertiary amine and represent, each independently, alkyl, substituted alkyl, cycloalkyl, substituted cycloalkyl, bicycloalkyl, substituted bicycloalkyl, aryl, substituted aryl, heterocyclyl, substituted heterocyclyl, or $R_1$, $R_2$ and $R_3$ or two of them form a cyclic system or together with an oxygen, nitrogen or sulphur atom form a heterocyclic system; preferably $R_1$, $R_2$ and $R_3$ form a cyclic or heterocyclic, and especially bicyclic or heterobicyclic system,

X represents a substituted or unsubstituted alkoxy, cycloalkoxy, substituted or unsubstituted alkylamino, dialkylamino, alkylarylamino, arylamino or diarylamino, preferably methoxy, and

Y represents a system of covalent bonds linking a carbon atom or carbon atoms and optionally heteroatoms such as oxygen, nitrogen or sulphur, forming the structures of a chain, ring, or chain and ring, linking the sulphonic residue to a triazine ring, excluding an azo linkage described in the prior art related to reactive dyes indicated above. In particular, Y represents an aliphatic chain linked to a triazine ring *via* a heteroatom such as nitrogen, oxygen or sulphur.

Preferably the aliphatic chain in the substituent Y is an aliphatic chain C2-C10, most preferably C2-C6.

Y can also represent an aromatic or heteroaromatic ring linked to a triazine ring *via* a heteroatom such as nitrogen, oxygen or sulphur. Preferably the ring is a 6-membered ring.

According to the invention a method of preparation of inner quaternary N-triazinylammonium salts of sulphonic acids of the above Formula 1, wherein $R_1$, $R_2$, $R_3$, X and Y are as defined above, comprises the steps wherein:

a) the sulphonic acid salt is treated with a chlorotriazine to form the corresponding triazine derivative,
b) the triazine derivative obtained in step (a) is treated with a tertiary amine

wherein the reaction is carried out in water or in a mixture of water with tetrahydrofuran or acetonitrile, for 2 hours to 14 days, at a nearly equimolar stoichiometry of substrates, and

step (b) may optionally be preceded by a step of isolation of the triazine derivative from the reaction medium.

The obtained product is filtered off, recrystallised from water and dried.

The term "nearly equimolar" stoichiometry of a reaction means that the reaction is carried out at a stoichiometry of substrates close to the equimolar one, such as from 0.8 to 1.2, and most preferably equimolar.

In the method of the invention an amino derivative sulphonic acid salt is preferably used as the sulphonic acid salt, and an aminotriazine is the triazine derivative obtained in step (a).

Preferably 2,4-dichloro-6-methoxy-1,3,5-triazine is used as a chlorotriazine in step (a), and N-methylmorpholine is used as a tertiary amine.

In the preferred embodiment, the method of the invention is carried out at a temperature of 0-10°C.

Also preferably, the product obtained by the method of the invention is in the form of a mixture with inorganic salts.

Optionally, the product is obtained in the form of mixed crystals formed with inorganic salts, especially with sodium, potassium or lithium chloride or sulphonate, or in the form of a mixture with these inorganic salts.

The invention relates also to the use of inner quaternary N-triazinylammonium salts of sulphonic acids of the Formula 1, wherein $R_1$, $R_2$, $R_3$, X and Y are as defined above, as condensation reagents in the condensation reactions, preferably in the reactions of peptide, ester or amide synthesis, and for protein modification.

Optionally, the N-triazinylammonium salts are used in the form of mixed crystals formed with inorganic salts, especially with sodium, potassium or lithium chloride or sulphonate, or in the form of a mixture with these inorganic salts.

The N-triazinylammonium salts according to the invention are characterised in that both salt-forming ionic fragments are joined together with a covalent bond and form an inner salt. An N-triazinylammonium cation is one of the elements forming the fragment bearing positive charge. The counterion of the N-triazinylammonium cation is the residue of sulphonic acid, which is linked via a series of covalent bonds of any length. The exchange of an anionic fragment for other anions, in particular, for anions reducing the salt stability or diminishing their synthetic usefulness, is hindered due to the presence of both ions in one molecule.

Depending on the conditions of the environment, the positive and negative charges compensate,

which results in the molecule of an inner salt that may be devoid of charge, but also may be positively or negatively charged depending on change in the environment conditions (pH of the environment). Thus, the ionic structure of the inner N-triazinylammonium salts is particularly beneficial. The inner salts can be used suitably both in the environment of organic solvents, in the aqueous organic medium, and even in aqueous environment. This makes it possible to use these salts for modifying of proteins. The ionic structure of by-products formed by the reactant molecule, especially the presence of sulphonic anion, is also advantageous, since this provides good solubility of intermediate products and by-products in water. This enables highly effective washing out of the impurities as well as the reagent excess after the condensation is completed. The above-mentioned advantages are also characteristic for mixed salts or mixtures formed from inner sulphonate salts with inorganic salts, especially with sodium, potassium or lithium chloride or sulphonate.

The method according to the invention is preparatively suitable, makes use of readily available and cost-effective reagents and proves its usefulness for a variety of triazine derivatives. This fact makes modification of N-triazinylammonium salts structure easy in the most suitable way with regard to the reaction conditions.

The method according to the invention is illustrated in the Examples below. The abbreviations present in Examples 10-24 and 27 represent: Boc = *tert*-butoxycarbonyl, Me = methyl, Phe = phenylalanine, Leu = leucine, Gly = glycine, Tyr = tyrosine, Z = benzyloxycarbonyl, Ala = alanine.

Example 1.

Sulphanilic acid (17.2 g, 0.1 mol) was added portionwise to a vigorously stirred and cooled suspension of sodium bicarbonate ($NaHCO_3$, 8.4 g, 0.1 mol) in a mixture of tetrahydrofuran (THF, 50 ml) and water (50 ml). After dissolution of the substrates, 2,4-dichloro-6-methoxy-1,3,5-triazine (DCMT, 18.0 g, 0.1 mol) was added portionwise at a rate allowing to keep the temperature of the mixture below 10°C. After reaction of DCMT is completed [as confirmed by disappearence of the triazine spot, having an $R_f$ = 0.6 on a chromatographic plate developed in a mobile phase with 100% $CHCl_3$ visualised with 4-(4-nitrobenzyl)pyridine], N-methylmorpholine (NMM, 11.1 ml, 0.1 mol) was added to the reaction mixture. After 24 hours, a white precipitate was formed and filtered off, recrystallised from water, and dried in the air to a constant weight.

N-[2-(4-Sulphonatephenylamino)-4-methoxy-1,3,5-triazin-6-yl]-4-methylmorpholinium inner salt (SPMT) pentahydrate (41.58 g, 88% yield) was obtained in the form of white crystals having the melting point (m.p.) of 103-105°C and the decomposition temperature of 262-264°C.

The results of the NMR analysis and elementary analysis of the obtained compound were as follows:

$^1H$ NMR ($D_2O$/NaOD) $\delta$ = 2.080 (s, 3H, $N^+$-C$\underline{H}_3$); 2.323 (m, 4H, C$\underline{H}_2$); 3.575 (t, J = 4.5 Hz, 4$\underline{H}$, $CH_2$); 3.724 (s, 3H, OC$\underline{H}_3$); 7.484-7.638 (m, 4H, $C_{Ar}\underline{H}$) [ppm],

Elementary analysis for $C_{15}H_{29}N_5O_{10}S$ (M = 471.51)

| | | | |
|---|---|---|---|
| Calculated: | C 38.13% | H 6.40% | N 14.82% |
| Found: | C 38.22% | H 6.19% | N 14.85%. |

Example 2.

Sulphanilic acid (5.90 g, 0.055 mol) was added portionwise to a vigorously stirred and cooled suspension of $NaHCO_3$ (4.90 g, 0.055 mol) in the mixture of acetonitrile (50 ml) and water (50 ml). After dissolution of the substrates, DCMT (10.00 g, 0.055 mol) was added portionwise at a rate allowing to keep the temperature of the mixture below 10°C. After reaction of DCMT is completed (what was ascertained as in Example 1), NMM (6.1 ml, 0.055 mol) was added to the reaction mixture. After 24 hours, the white precipitate was filtered off, recrystallised from water and dried in the air to a constant weight.

N-[2-(4-Sulphonatephenylamino)-4-methoxy-1,3,5-triazin-6-yl]-4-methylmorpholinium inner salt (SPMT) pentahydrate (22.04 g, 84% yield) was obtained in the form of white crystals having the $^1H$ NMR characteristics identical as for the product obtained in Example 1.

Example 3.

Sodium 2-(4-aminophenylsulphonate)-4-chloro-6-methoxy-1,3,5-triazinyl (8.40 g, 0.025 mol) was dissolved in water, and afterwards the obtained mixture was cooled in an ice bath. NMM (2.75 ml, 0.025 mol) was added dropwise to the cooled mixture. As it was added an intense heat release was observed. Subsequently the reaction mixture was warmed to room temperature. A spontaneous precipitation of white solid was observed. After 24 hours, the solid was filtered off and dried in the air.

N-[2-(4-Sulphonatephenylamino)-4-methoxy-1,3,5-triazin-6-yl]-4-methylmorpholinium inner salt (SPMT) pentahydrate (10.42 g, 92% yield) was obtained in the form of white crystals having the $^1H$ NMR spectrum identical with the spectrum of the product obtained in Example 1.

Example 4.

A synthesis of an N-triazinylammonium salt was carried out for 10 days as in Example 1, using methanilic acid (1.72 g, 0.010 mol), DCMT (1.80 g, 0.010 mol), NMM (1.10 ml, 0.010 mol) and $NaHCO_3$ (0.84 g, 0.010 mol). After 10 days, the formed solid was filtered off, recrystallised from water, filtered off again, and dried in the air to a constant weight. The product N-[2-(3-sulphonatephenylamino)-4-methoxy-1,3,5-triazin-6-yl]-4-methylmorpholinium inner salt (SMMT) trihydrate (2.69 g, 62%

yield) was obtained in the form of white crystals having the decomposition temperature of 243-244°C. The results of NMR analysis and elementary analysis of the obtained compound are as follows:
$^1$H NMR ($D_2O$/NaOD) δ = 2.224 (s, 3H, $N^+$-C$\underline{H}_3$); 2.469 (m, 4H, C$\underline{H}_2$); 3.720 (t, J = 4.5 Hz, 4H, C$\underline{H}_2$); 3.829 (s, 3H, $OCH_3$); 7.491-7.545 (m, 2H, $C_{Ar}\underline{H}$); 7.630-7.705 (m, 1H, $C_{Ar}\underline{H}$); 8.043-8.075 (m, 1H, $C_{Ar}\underline{H}$) [ppm]
Elementary analysis for $C_{15}H_{25}N_5O_8S$ (M = 435.31)

| | | | |
|---|---|---|---|
| Calculated: | C 41.37% | H 5.79% | N 16.08% |
| Found: | C 40.98% | H 5.40% | N 16.00% |

Example 5.

A synthesis of an N-triazinylammonium salt was carried out for 24 hours as in Example 1, using orthanilic acid (1.72 g, 0.010 mol), DCMT (1.80 g, 0.010 mol), NMM (1.10 ml, 0.010 mol) and $NaHCO_3$ (0.84 g, 0.010 mol). After 24 hours, the white precipitate was filtered off, recrystallised from water, filtered off again, and dried in the air. N-[2-(2-Sulphonatephenylamino)-4-methoxy-1,3,5-triazin-6-yl]-4-methylmorpholinium inner salt (SOMT) trihydrate (3.11 g, 72% yield) was obtained in the form of white crystals having the m.p. of 222-224°C and the decomposition temperature of 243-244°C.

The results of NMR analysis and elementary analysis of the obtained compound are as follows:
$^1$H NMR ($D_2O$/NaOD) δ = 2.223 (s, 3H, $N^+$-C$\underline{H}_3$); 2.467 (m, 4H, $CH_2$); 3.720 (t, J = 4.5 Hz, 4H, C$\underline{H}_2$); 3.865 (s, 3H, OC$\underline{H}_3$) ; 7.223 (dt, J = 7.5 Hz, J = 1.25 Hz, 1H, $C_{Ar}\underline{H}$); 7.548 (ddt, J = 7.5 Hz, J = 1.25 Hz, J = 0.75 Hz 1H, $C_{Ar}\underline{H}$); 7.837 (dd, J = 7.5 Hz, J = 1.25 Hz, 1H, $C_{Ar}\underline{H}$); 8.095 (dd, J = 7.5 Hz, J = 0.75 Hz, 1H, $C_{Ar}\underline{H}$) [ppm]
Elementary analysis for $C_{15}H_{25}N_5O_8S$ (M = 435.31)

| | | | |
|---|---|---|---|
| Calculated: | C 41.37% | H 5.79% | N 16.08% |
| Found: | C 41.40% | H 5.01% | N 16.07% |

Example 6.

A synthesis of an N-triazinylammonium salt was carried out for 24 hours as in Example 1, using 8-aminonaphthalene-2-sulphonic acid (marketed under the trade name of 1,7-Cleve's acid, 1.24 g, 0.0055 mol), DCMT (1.00 g, 0.0055 mol), NMM (0.6 ml, 0.0055 mol), and $NaHCO_3$ (0.46 g, 0.0055 mol). After 24 hours, the white precipitate was filtered off, recrystallised from water, filtered off again, and dried in the air. N-[2-(2-Sulphonatenaphth-8-ylamino)-4-methoxy-1,3,5-triazin-6-yl]-4-methylmorpholinium inner salt (SCLMT) dihydrate in the form of a co-precipitate with NaCl (2.54 g, 88% yield) was obtained in the form of white crystals having the decomposition temperature of 270-271°C. The results of NMR analysis and elementary analysis of the obtained compound are as follows:
$^1$H NMR ($D_2O$/NaOD) δ = 2.227 (s, 3H, $N^+$-C$\underline{H}_3$); 2.473 (t, J = 4.5 Hz, 4H, C$\underline{H}_2$); 3.722 (t, J = 4.5 Hz, 4H, C$\underline{H}_2$); 3.747 (s, 3H, OC$\underline{H}_3$); 7.626-7.709 (m, 2H, $C_{Ar}\underline{H}$); 7.828-7.968 (m, 2H, $C_{Ar}\underline{H}$); 8.098 (d, J = 9 Hz, 1H, $C_{Ar}\underline{H}$); 8.421 (s, 1H, $C_{Ar}\underline{H}$); [ppm]
Elementary analysis for $C_{19}H_{25}N_5O_7S$ x NaCl (M = 525.95)

| | | | |
|---|---|---|---|
| Calculated: | C 43.39% | H 4.79% | N 13.32% |
| Found: | C 43.66% | H 4.98% | N 13.20% |

Example 7.

A synthesis of an N-triazinylammonium salt was carried out for 24 hours as in Example 1, using 4-aminonaphthyl-1-sulphonic acid (marketed under the trade name of naphthionic acid, 1.24 g, 0.0055 mol), DCMT (1.00 g, 0.0055 mol), NMM (0.6 ml, 0.0055 mol) and $NaHCO_3$ (0.46 g, 0.0055 mol). After 24 hours, the white precipitate was filtered off, recrystallised from water and dried in the air. N-[2-(1-Sulphonatenaphth-4-ylamino)-4-methoxy-1,3,5-triazin-6-yl]-4-methylmorpholinium inner salt (SNMT) in the form of a co-precipitate with NaCl (2.04 g, 75% yield) was obtained in the form of white crystals having the decomposition temperature of 269-270°C.

The results of NMR analysis and elementary analysis of the obtained compound are as follows:
$^1$H NMR ($D_2O$/NaOD) δ = 2.230 (s, 3H, $N^+$-C$\underline{H}_3$); 2.474 (m, 4H, C$\underline{H}_2$); 3.725 (t, J = 4.5 Hz, 4H, C$\underline{H}_2$); 3.787 (s, 3H, OC$\underline{H}_3$); 7.623-7.798 (m, 3H, $C_{Ar}\underline{H}$); 8.134 (d, J = 8.5 Hz, 2H, $C_{Ar}\underline{H}$); 8.668 (d, J = 9 Hz, 1H, $C_{Ar}\underline{H}$); [ppm]
Elementary analysis for $C_{19}H_{21}N_5O_5S$ x NaCl (M = 489.92)

| | | | |
|---|---|---|---|
| Calculated: | C 46.58% | H 4.32% | N 14.29% |
| Found: | C 46.36% | H 4.40% | N 14.08% |

Example 8.

A synthesis of an N-triazinylammonium salt was carried out for 24 hours as in Example 1, using 4-aminonaphthyl-1-sulphonic acid (marketed under the trade name of naphthionic acid, 1.24 g, 0.0055 mol), DCMT (1.00 g, 0.0055 mol), NMM (0.6 ml, 0.0055 mol) and $KHCO_3$ (0.55 g, 0.0055 mol). After 24 hours, the white precipitate was filtered off, recrystallised from water and dried in the air. N-[2-(1-sulphonatenaphth-4-ylamino)-4-methoxy-1,3,5-triazin-6-yl]-4-methylmorpholinium inner salt (SNMT) in the form of a co-precipitate with KCl (2.00 g, 80% yield) was obtained in the form of white crystals having the decomposition temperature of 269-270°C.

The results of NMR analysis and elementary analysis of the obtained compound are as follows:
$^1$H NMR ($D_2O$/NaOD) δ = 2.230 (s, 3H, $N^+$-C$\underline{H}_3$); 2.474 (m, 4H, C$\underline{H}_2$); 3.725 (t, J = 4.5 Hz, 4H, C$\underline{H}_2$); 3.787 (s, 3H, OC$\underline{H}_3$); 7.623-7.798 (m, 3H, $C_{Ar}\underline{H}$); 8.134 (d, J = 8.5

Hz, 2H, $C_{Ar}\underline{H}$); 8.668 (d, J = 9 Hz, 1H, $C_{Ar}\underline{H}$); [ppm]
Elementary analysis for $C_{19}H_{21}N_5O_5S$ x 1/3 KCl (M = 456.32)

| | | | |
|---|---|---|---|
| Calculated: | C 50.01% | H 4.64% | N 15.35%. |
| Found: | C 50.31% | H 4.78% | N 14.87% |

Example 9.

A synthesis of an N-triazinylammonium salt was carried out for 24 hours as in Example 1, using taurine (0.62 g, 5.5 mmol), DCMT (1.00 g, 5.5 mmol), NMM (0.6 ml, 5.5 mmol) and $NaHCO_3$ (0.52 g, 5.5 mmol). After 24 hours, brine (10 ml) was added and then the solution was concentrated to dryness. The residue was extracted with methanol (3 x 10 ml). Methanol was stripped off from the combined extracts *in vacuo,* and the residue was crystallised from acetone/ methanol. The precipitate was filtered off and dried in the air. 2-[(2-Sulphonate-ethylamino)-4-methoxy-1,3,5-triazin-6-yl]-4-methylmorpholinium inner salt hydrate in the mixture with sodium chloride (1.75 g, 68% yield) was obtained in the form of white crystals having the m.p. of 116-118°C. The results of NMR analysis and elementary analysis of the obtained compound are as follows:
$^1H$ NMR ($D_2O$) $\delta$ = 3.165 (t, J = 7 Hz, 1H, $CH_2$); 3.229 (t, J = 7 Hz, 1H, $C\underline{H}_2$); 3.447;3.487 (s, 3H, $C\underline{H}_3$); 3.720-3.935 (m, 6H, $C\underline{H}_2$); 4.019;4.074 (s, 3H, $C\underline{H}_3$); 4.129 (m, 2H, $C\underline{H}_2$); 4.483-4.655 (m, 2H, $C\underline{H}_2$); [ppm]
$^1H$ NMR ($D_2O$/NaOD) $\delta$ = 2.229 (s, 3H, $N^+$-$C\underline{H}_3$); 2.479 (m, 4H, $C\underline{H}_2$) ; 3.161 (t, J = 6.75 Hz, 2H, $C\underline{H}_2$) ; 3.694 (t, J = 7.5 Hz, 2H, $C\underline{H}_2$); 3.722 (t, J = 5 Hz, 4H, $C\underline{H}_2$); 3.833 (s, 3H, $OC\underline{H}_3$); [ppm]
Elementary analysis for $C_{11}H_{21}N_5O_6S$ x 2 NaCl (M = 468.27)

| | | | |
|---|---|---|---|
| Calculated: | C 28.22% | H 4.52% | N 14.96% |
| Found: | C 28.24% | H 4.54% | N 15.01% |

Example 10.

In a round-bottomed flask, Boc-Phe-OH (0.53 g, 0.002 mol), H-LeuOMe x HCl (0.364 g, 0.002 mol), SPMT x $5H_2O$ (0.944 g, 0.002 mol) were dissolved in DMF (5 ml) and stirred using a magnetic stirrer. The reaction was carried out for 24 hours, during which the presence of the condensation reagent and the triazine ester was being checked. After disappearance of the triazine spot [visualised with 4-(4-nitrobenzyl)pyridine], 100 ml of a mixture of saturated solutions of sodium chloride (50 ml) and sodium bicarbonate (50 ml) were added dropwise to the reaction mixture. The precipitate was filtered off and dried in the air.

Boc-Phe-Leu-OMe (0.745 g, 95% yield) was obtained in the form of a white powder having the m.p. of 103-104°C (lit. value of 104-105°C), the specific rotation $[\alpha]^{20}_{589}$ = (-) 32.0 (c = 0.4, MeOH) [lit. value $[\alpha]^{20}_{589}$ = (-) 24.9 (MeOH)].

The results of NMR analysis of the obtained compound are as follows:

$^1H$ NMR ($CDCl_3$) $\delta$ = 0.899 (t, J = 5.5 Hz, 6H, $C\underline{H}_3$); 1.414 (s, 9H, $C\underline{H}_3$); 1.439-1.692 (m, 3H, $C\underline{H}_2$, $C\underline{H}$); 3.071 (d, J = 7 Hz, 2H, $C\underline{H}_2$) ; 3.691 (s, 3H, $OC\underline{H}_3$) ; 4.343 (q, J = 7.5 Hz, 1H, $C\underline{H}$); 4.516-4.624 (m, 1H, $C\underline{H}$); 4.987 (m, 1H, $N\underline{H}$); 6.242 (d, J = 8 Hz, 1H, $N\underline{H}$); 7.166-7.347 (m, 5H, $C_{Ar}\underline{H}$); [ppm].

Example 11.

The synthesis was carried out as in Example 10, using Boc-Gly-OH (0.227 g, 0.0013 mol), H-Phe-LeuOMe x HCl (0.439 g, 0.0013 mol) and SPMT x $5H_2O$ (0.615 g, 0.0013 mol). Boc-Gly-Phe-Leu-OMe (0.490 g, 87% yield) was obtained in the form of a white powder having the m.p. of 116-118°C (lit. value 113-114°C), the specific rotation $[\alpha]^{20}_{589}$ = (-) 27.6 (c = 0.865, MeOH) [lit. value $[\alpha]^{20}_{589}$ = (-) 20.5 (MeOH)].

The results of NMR analysis of the obtained compound are as follows:

$^1H$ NMR ($CDCl_3$) $\delta$ = 0.885 (d, J = 4.75 Hz, 6H, $C\underline{H}_3$); 1.429 (s, 9H, $C\underline{H}_3$); 1.454-1.608 (m, 3H, $C\underline{H}_2$, $C\underline{H}$); 3.092 (dAB, J = 7 Hz, 2H, $C\underline{H}_2$); 3.695 (s, 3H, $OC\underline{H}_3$); 3.769 (d, J = 6 Hz, 2H, $C\underline{H}_2$); 4.465-4.570 (m, 1H, $C\underline{H}$); 4.701 (q, J = 7 Hz, 1H, $C\underline{H}$); 5.046-5.150 (m, 1H, $N\underline{H}$); 6.275-6.387 (m, 1H, $N\underline{H}$); 6.714 (d, J = 7 Hz, 1H, $N\underline{H}$); 7.176-7.347 (m, 5H, $C_{Ar}\underline{H}$); [ppm].

Example 12.

The synthesis was carried out as in Example 10, using Boc-Gly-OH (0.166 g, 0.00095 mol), H-Gly-Phe-LeuOMe x HCl (0.366 g, 0.00095 mol), SPMT x $5H_2O$ (0.448 g, 0.00095 mol).

Boc-Gly-Gly-Phe-Leu-OMe (0.498 g, 98% yield) was obtained in the form of a white powder having the m.p. of 126-128°C (lit. value of 125-127°C), the specific rotation $[\alpha]^{20}_{589}$ = (-) 26.1 (c= 0.42, MeOH). The results of NMR analysis of the obtained compound are as follows:

$^1H$ NMR (acetone-$d_6$) $\delta$ = 0.907 (t, J = 6.25 Hz, 6H, $C\underline{H}_3$); 1.423 (s, 9H, $C\underline{H}_3$) ; 1.486-1.747 (m, 3H, $C\underline{H}_2$, $C\underline{H}$); 2.918 (AB, 2H, $C\underline{H}_2$); 3.661 (s, 3H, $OC\underline{H}_3$); 3.741 (d, J = 5.75 Hz, 2H, $C\underline{H}_2$); 3.789 (d, J = 5.5 Hz, 2H, $C\underline{H}_2$); 4.402-4.520 (m, 1H, $C\underline{H}$); 4.701 (dt, J

= 5 Hz, J = 8 Hz, 1H, CH); 6.378 (m, 1H, NH) ; 7.143-7.334 (m, 5H, $C_{Ar}H$, NH); 7.421-7.572 (m, 2H, $C_{Ar}H$); 7.679 (m, 1H, NH); [ppm].

Example 13.

The synthesis was carried out as in Example 10, using Boc-Tyr(OBoc)-OH (0.310 g, 0.00092 mol), H-Gly-Gly-Phe-LeuOMe x HCl (0.470 g, 0.00092 mol), SPMT x $5H_2O$ (0.435 g, 0.00106 mol).

Boc-Tyr(OBoc)-Gly-Gly-Phe-Leu-OMe (0.550 g, 75% yield) was obtained in the form of a white powder having the m.p. of 140-142°C and the specific rotation $[\alpha]_{589}^{20}$ = (−) 9.6 (c = 1.01, MeOH). The results of NMR analysis of the obtained compound are as follows:

$^1$H NMR (acetone-$d_6$) δ = 0.903 (t, J = 7.25 Hz, 6H, $CH_3$); 1.360 (s, 18H, $CH_3$); δ = 1.506-1.764 (m, 3H, $CH_2$, CH) ; 2.831-3.293 (m, 4H, $CH_2$); 3.654 (s, 3H, $OCH_3$); 3.704-3.876 (m, 4H, $CH_2$) ; 4.310 (q, J = 7.25 Hz, 1H, CH) ; 4.473 (sx, J = 4.75 Hz, 1H, CH); 4.663 (sx, J = 4.5 Hz, 1H, CH); 6.344 (d, J = 7.25 Hz, 1H, NH); 6.744 (d, J = 8.5 Hz, 1H, $C_{Ar}H$); 7.072 (d, J = 8.5 Hz, 1H, $C_{Ar}H$); 7.145-7.353 (m, 3H, $C_{Ar}H$, NH); 7.568-7.719 (m, 2H, $C_{Ar}H$); 7.679 (m, 1H, NH); 7.942 (s, 1H, NH); 8.062 (s, 1H, NH); [ppm].

Example 14.

In a round-bottomed flask, Z-Phe-OH (0.299 g, 0.001 mol) and H-Phe-OMe x HCl (0.216 g, 0.001 mol) were dissolved in 20 ml of 1 N $NaHCO_3$. SPMT pentahydrate (0.471 g, 0.001 mol) was then added to the reaction mixture. The reaction was carried out at room temperature. During the reaction, a formation of white precipitate was observed. After disappearance of the triazine spot [$R_f$ = 0.0, 100% chloroform, visualised with 4-(4-nitrobenzyl)pyridine], the formed solid was filtered off and dried in the air. Z-Phe-Phe-OMe (0.353 g, 77% yield) was obtained in the form of a white powder, having the m.p. of 143-144°C (lit. 146-148°C). The results of NMR analysis of the obtained compound are as follows:

$^1$H NMR (acetone-$d_6$) δ = 3.040 (m, $CH_2$, 4H); 3.668 (s, $CH_3$, 3H); 4.391 (q, J = 6.5 Hz, CH, 1H); 4.772 (dt, J = 7.5 Hz, J = 6.5 Hz, CH, 1H); 5.072 (s, $CH_2$, 2H); 5.235 (m, NH, 1H); 6.205 (d, J = 7.5 Hz, NH, 1H); 6.930-7.000 (m, $C_{Ar}H$, 2H), 7.077-7.395 (m, $C_{Ar}H$, 13H); [ppm].

Example 15.

The synthesis was carried out as in Example 14, at 0°C.

Z-Phe-Phe-OMe (0.233 g, 51% yield) was obtained in the form of a white powder. The results of analysis of the obtained compound were analogous to those of the compound obtained in Example 14.

Example 16.

In a flask sodium N-[2-(4-aminophenylsulphonate)-4-chloro-6-methoxy-1,3,5-triazinyl] (0.337 g, 0.001 mol) was dissolved in water, NMM (0.11 ml, 0.001 mol) was added, and the flask was placed in an ice bath. After disappearance of sodium N-[2-(4-aminophenylsulphonate)-4-chloro-6-methoxy-1,3,5-triazinyl] ($R_f$ = 0.1, 100% chloroform), the mixture was warmed to room temperature, and then Z-Phe-OH (0.299 g, 0.001 mol) and $NaHCO_3$ (0.084 g, 0.001 mol) were added to the reaction mixture. After disappearance of the SPMT spot [$R_f$ = 0.0, 100% chloroform, visualised with 4-(4-nitrobenzyl)-pyridine], H-Phe-OMe x HCl (0.216 g, 0.001 mol) and $NaHCO_3$ (0.084 g, 0.001 mol) were added to the reaction mixture. A formation of white precipitate was observed. After disappearance of the triazine ester ($R_f$ = 0.1, 100% chloroform), the solid was filtered off and dried in the air. Z-Phe-Phe-OMe (0.202 g, 44% yield) was obtained in the form of a white powder. The results of NMR analysis of the obtained compound are analogous to those for the compound obtained in Example 14.

Example 17.

In a flask, sodium N-[2-(4-aminophenylsulphonate)-4-chloro-6-methoxy-1,3,5-triazinyl] (0.337 g, 0.001 mol) was dissolved in water, NMM (0.11 ml, 0.001 mol) was added and the flask was placed in an ice bath. After disappearance of the spot of sodium N-[2-(4-aminophenyl-sulphonate)-4-chloro-6-methoxy-1,3,5-triazinyl] [$R_f$ = 0.1, 100% chloroform, visualised with 4-(4-nitrobenzyl) pyridine], Z-Phe-OH (0.299 g, 0.001 mol) and $NaHCO_3$ (0.084 g, 0.001 mol) were added to the reaction mixture. After disappearance of SPMT ($R_f$ = 0.0, 100% chloroform), H-Phe-OMe x HCl (0.216 g, 0.001 mol) and $NaHCO_3$ (0.084 g, 0.001 mol) were added to the reaction mixture. A formation of white precipitate was observed. After disappearance of the triazine ester ($R_f$ = 0.1, 100% chloroform), the solid was filtered off and dried in the air.

Z-Phe-Phe-OMe (0.168 g, 33% yield) was obtained in the form of a white powder. The results of NMR analysis of the obtained compound were analogous to those for the compound obtained in Example 14.

Example 18.

In a flask, sodium N-[2-(4-aminophenylsulphonate)-4-chloro-6-methoxy-1,3,5-triazinyl] (0.337 g, 0.001 mol) was dissolved in water, and NMM (0.11 ml, 0.001 mol), Z-Phe-OH (0.299 g, 0.001 mol) and $NaHCO_3$ (0.084 g, 0.001 mol) were added. The flask was placed in an ice bath. After disappearance of the spot of sodium N-[2-(4-aminophenylsulphonate)-4-chloro-6-methoxy-1,3,5-triazinyl] [$R_f$ = 0.1, 100% chloroform, visualised with 4-(4-nitrobenzyl)pyridine], H-Phe-OMe x HCl (0.216 g, 0.001 mol) and $NaHCO_3$ (0.084 g, 0.001 mol) were added to the reaction mixture. A formation of white precipitate was observed. After disappearance of the triazine ester, the solid was filtered off and dried in the air.

Z-Phe-Phe-OMe (0.068 g, 15% yield) was obtained in the form of a white powder. The results of NMR analysis of the obtained compound were analogous to those for the compound obtained in Example 14.

Example 19.

A round-bottomed flask was charged with Z-Ala-OH (0.223 g, 0.001 mol), H-Ala-OMe x HCl (0.140 g, 0.001 mol), SPMT x $5H_2O$ (0.471 g, 0.001 mol) and $NaHCO_3$ (0.168 g, 0.002 mol). Water (10 ml) was added to the flask and the mixture was stirred using a magnetic stirrer. During the reaction, a formation of white precipitate was observed. After 5 hours, the precipitate was filtered off and dried in the air. Z-Ala-Ala-OMe (0.278 g, 90% yield) was obtained in the form of a white powder, having the m.p. of 93-94°C (lit. 106-108°C). The results of NMR analysis of the obtained compound are as follows:

$^1H$ NMR (acetone-$d_6$) $\delta$ = 1.341 (d, J = 7 Hz, C$\underline{H}_3$, 3H); $\delta$ = 1.380 (d, J = 7 Hz, C$\underline{H}_3$, 3H); 3.673 (s, C$\underline{H}_3$, 3H); 4.240 (dp, J = 7.25 Hz, J = 2 Hz, C$\underline{H}$, 1H); 4.435 (dp, J = 7.5 Hz, J = 1 Hz, C$\underline{H}$, 1H); 5.077 (d, J = 1.5 Hz, C$\underline{H}_2$, 2H); 6.482 (m, N$\underline{H}$, 1H); 7.267-7.417 (m, $C_{Ar}\underline{H}$, 5H); 7.536 (m, N$\underline{H}$, 1H) [ppm].

Example 20.

In a flask, Z-Ala-OH (0.223 g, 0.001 mol), NMM (0.11 ml, 0.001 mol) and SPMT x $5H_2O$ (0.471 g, 0.001 mol) were dissolved in DMF (2 ml). After dissolving, a formation of white precipitate was observed. After 30 minutes the slurry was added to 10 ml of water containing H-Ala-OMe x HCl (0.140 g, 0.001 mol) and $NaHCO_3$ (0.084 g, 0.001 mol). After adding the slurry of the triazine ester, the reaction mixture was stirred for 30 minutes, followed by adding 50 ml of 1 N $NaHCO_3$. The precipitate was filtered off and dried in the air.

Z-Ala-Ala-OMe (0.250 g, 81% yield) was obtained in the form of a white powder. The results of analysis of the obtained compound were analogous to those for the compound obtained in Example 19.

Example 21.

A round-bottomed flask was charged with Z-Ala-OH (0.223 g, 0.001 mol), H-Phe-OMe x HCl (0.216 g, 0.001 mol), SPMT x $5H_2O$ (0.471 g, 0.001 mol) and $NaHCO_3$ (0.168 g, 0.002 mol). Water (10 ml) was added to the flask and the mixture was stirred using a magnetic stirrer. During the reaction, a formation of white precipitate was observed. After 5 hours, the solid was filtered off and dried in the air. Z-L-Ala-L-Phe-OMe (0.347 g, 90% yield) was obtained in the form of a white powder having the m.p. 88-89°C (lit. 99-101°C), the specific rotation $[\alpha]_{589}^{20}$ = (−) 12.1 (c = 1.38, MeOH) [lit. $[\alpha]_{589}^{20}$ = (−) 14.1 (MeOH)]. The results of NMR analysis of the obtained compound are as follows:

$^1H$ NMR (acetone-$d_6$) $\delta$ = 1.274 (d, J = 7 Hz, C$\underline{H}_3$, 3H); 3.054 (m, C$\underline{H}_2$, 2H); 3.636 (s, C$\underline{H}_3$, 3H); 4.206 (p, J = 7.5 Hz, C$\underline{H}$, 1H) ; 4.682 (dt, J = 7.5 Hz, J = 6 Hz, C$\underline{H}$, 1H) ; 5.045 (s, C$\underline{H}_2$, 2H); 6.457 (m, N$\underline{H}$, 1H); 7.121-7.451 (m, $C_{Ar}\underline{H}$, 10H); 7.420 (d, J = 7.5 Hz, N$\underline{H}$, 1H); [ppm].

Example 22.

In a flask, Z-Ala-OH (0.223 g, 0.001 mol), NMM (0.11 ml, 0.001 mol) and SPMT x $5H_2O$ (0.471 g, 0.001 mol) were dissolved in DMF (2 ml). Just after complete dissolution, a formation of white precipitate was observed. After 30 minutes, the slurry was added to 10 ml of water containing H-Phe-OMe x HCl (0.216 g, 0.001 mol) and $NaHCO_3$ (0.084 g, 0.001 mol). After adding the slurry, stirring was continued for 30 minutes, followed by adding 50 ml of 1 M $NaHCO_3$. The precipitate was filtered off and dried in the air.

Z-Ala-Phe-OMe (0.309 g, 80% yield) was obtained in the form of a white powder. The results of analysis of the obtained compound were analogous to those for the compound obtained in Example 21.

Example 23.

A round-bottomed flask was charged with Z-Ala-OH (0.223 g, 0.001 mol), H-Leu-OMe x HCl (0.180 g, 0.001 mol), SPMT x $5H_2O$ (0.471 g, 0.001 mol) and $NaHCO_3$ (0.168 g, 0.002 mol). Water (10 ml) was added to the flask and the mixture was stirred using a magnetic stirrer. During the reaction a formation of white precipitate was observed. After 5 hours, the solid was filtered off and dried in the air. Z-Ala-Leu-OMe (0.306 g, 88% yield) was obtained in the form of a white powder having the melting point of 81-82°C (lit. 71-73°C) and the specific rotation $[\alpha]_{589}^{20}$ = (−) 11.4 (c = 1.22, MeOH) [lit. value $[\alpha]_{589}^{20}$ = (−) 13.28 (MeOH)]. The results of NMR analysis of the obtained compound are as follows:

$^1H$ NMR (acetone-$d_6$) $\delta$ = 0.885 (m, C$\underline{H}_3$, 6H); 1.320 (d, J = 7 Hz, C$\underline{H}_3$, 3H); 1.454-1.777 (m, C$\underline{H}_2$, CH, 3H); 3.645 (s, C$\underline{H}_3$, 3H); 4.242 (dq, J = 7.25 Hz, J =

2.5 Hz, CH, 1H); 4.436-4.543 (m, CH, 1H); 5.053 (s, $CH_2$, 2H); 6.474 (m, NH, 1H); 7.237-7.372 (m, $C_{Ar}H$, 5H); 7.458 (d, J = 8 Hz, NH, 1H); [ppm].

Example 24.

In a flask, Z-Ala-OH (0.223 g, 0.001 mol), NMM (0.11 ml, 0.001 mol), SPMT x $5H_2O$ (0.471 g, 0.001 mol) were dissolved in DMF (2 ml). After dissolution, a formation of white precipitate was observed. After 30 minutes, the slurry was added to 10 ml of water containing H-Leu-OMe x HCl (0.180 g, 0.001 mol) and $NaHCO_3$ (0.084 g, 0.001 mol). After adding the slurry, stirring was continued during 30 minutes, followed by adding 50 ml of 1 M $NaHCO_3$. The precipitate was filtered off and dried in the air.

Z-Ala-Leu-OMe (0.286 g, 82% yield) was obtained in the form of a white powder. The results of analysis of the obtained compound were analogous to those for the compound obtained in Example 23.

Example 25.

In a flask, p-methoxybenzoic acid (0.152 g, 0.001 mol), p-toluidine (0.107 g, 0.001 mol), SPMT x $5H_2O$ (0.471 g, 0.001 mol) were dissolved in DMF (2 ml) and stirred using a magnetic stirrer. After 24 hours, a mixture of 25 ml of 1 N solution of $NaHCO_3$ and 25 ml of saturated sodium chloride solution were added to the reaction solution. The precipitate was filtered off, washed with 25 ml of 1 N hydrochloric acid, 25 ml of water, and dried in the air to a constant weight.

4-Methoxy-N-[4-methylphenyl]-benzamide (0.215 g, 89% yield) was obtained in the form of a white powder, having the melting point of 150-152°C (lit. 156-157°C). The results of NMR analyses of the obtained compound are as follows:

$^1H$ NMR ($CDCl_3$): δ = 2.332 (s, $C_{Ar}$-$CH_3$, 3 H); 3.860 (s, O-$CH_3$, 3 H); 6.949 (d, $J_{H-H}$ = 8.75 Hz, CHar, 2 H); 7.154 (d, $J_{H-H}$ = 8.5 Hz, $CH_{Ar}$, 2 H); 7.504 (d, $J_{H-H}$ = 8.5 Hz, $CH_{Ar}$, 2 H); 7.762 (s, NH, 1 H); 7.827 (d, $J_{H-H}$ = 8.75 Hz, $CH_A$, 2 H);

Example 26.

In a flask, p-methoxybenzoic acid (0.152 g, 0.001 mol), p-toluidine (0.107 g, 0.001 mol), SPMT x $5H_2O$ (0.471 g, 0.001 mol) were dissolved in the mixture of THF (5 ml) and water (5 ml) and stirred using a magnetic stirrer. After complete dissolution of the substrates, stirring was continued for 30 minutes. THF was evaporated *in vacuo*. A precipitate was filtered off from the residue. The precipitate was dissolved in ethyl acetate (10 ml) and washed successively with 3 x 10 ml of 1 N HCl, 3 x 10 ml of water, 3 x 10 ml of 1 N $NaHCO_3$, 3 x 10 of water and 1 x 10 ml of brine. The organic phase was dried over magnesium sulphate, filtered, and the filtrate was concentrated *in vacuo* to dryness. The residue was dried in desiccator.

4-Methoxy-N-[4-methylphenyl]-benzamide (0.112 g, 46% yield) was obtained. The results of analysis of the obtained compound were analogous to those for the compound obtained in Example 25.

Example 27.

In a flask, Z-Phe-OH (0.299 g, 0.001 mol) and SPMT x $5H_2O$ (0.471 g, 0.001 mol) were dissolved in a mixture of DMF (2 ml) and MeOH (0.25 ml) and stirred using a magnetic stirrer. Stirring was continued for 24 hours, followed by adding toluene (20 ml) and concentrating to dryness *in vacuo.* A mixture of ethyl acetate (20 ml) and water (20 ml) was added to the dry residue. The organic phase was separated, washed successively with 2 x 5 ml of water, 3 x 5 ml of 1 N solution of $NaHCO_3$, 3 x 5 ml of water and 1 x 5 ml of saturated NaCl solution. Then ethyl acetate was removed *in vacuo,* and the obtained product was dried in the air to a constant weight.

N-Benzyloxycarbonyl-L-phenylalanine methyl ester (0.204 g, 65% yield) was obtained in the form of an oil.

The results of NMR analyses are as follows:

$^1H$ NMR (acetone-$d_6$) δ= 3.090 (m, $CH_2$, 2H); 3.674 (s, $CH_3$, 3H); 4.496 (dt, J = 6.25 Hz, J = 8.75 Hz, CH, 1H); 5.027 (s, $CH_2$, 2H); 5.592 (m, NH, 1H); 7.183-7.390 (m, $C_{Ar}H$, 10H) [ppm].

Example 28

N-(2-(4-Aminophenylsulphonate)-4-methoxy-1,3,5-triazin-6-yl)-4-methylmorpholinium was used to modify lysozyme with carboxyfluorescein. To a solution of a mixture of carboxyfluorescein isomers (0.023 g; 6 x $10^{-5}$ mol) in DMF (0.5 ml), N-(2-(4-aminophenylsulphonate)-4-methoxy-1,3,5-triazin-6-yl)-4-methylmorpholinium (0.028 g; 6 x $10^{-5}$ mol) was added along with a catalytic amount of NMM. The resulting mixture was added dropwise to a solution of lysozyme (0.1 g; 6 x $10^{-6}$ mol) dissolved in 0.1 N solution of sodium bicarbonate (5 ml). During addition, formation of a characteristically coloured red-brown precipitate was observed. After adding was completed, the precipitate was separated by centrifugation. The obtained precipitate was washed with water (5 ml), and centrifuged again. After freeze-drying, 35 mg of a crude product in the form of red-brown solid was obtained. The obtained product was then purified by gel filtration on a bed of Sephadex G-25 (column 14 mm x 900 mm), using 0.1 N solution of $Na_2CO_3$ as a mobile phase. Two fractions were collected, i.e., the fraction B1 having $R_t$ = 150-190 minutes, and the fraction B2 having $R_t$ = 360-380 minutes. After freeze-drying, 12 mg of the fraction B1 and 9 mg of the fraction B2 were obtained.

Electrophoresis on an agarose gel (Lysozyme $R_f$ = -0.2; carboxyfluorescein $R_f$ = 0.6):

Fraction B1 $R_f$ = 0.3.
Fraction B2 $R_f$ = 0.1.

Example 29

N-(2-(4-Aminophenylsulphonate)-4-methoxy-1,3,5-triazin-6-yl)-4-methylmorpholinium was used to modify lysozyme with carboxyfluorescein. To a solution of a mixture of carboxyfluorescein isomers (0.023 g; 6 x $10^{-5}$ mol) in 0.5 ml of DMF N-(2-(4-aminophenylsulphonate)-4-methoxy-1,3,5-triazin-6-yl)-4-methylmorpholinium (0.028 g, 6 x $10^{-5}$ mol) was added along with a catalytic amount of NMM. The resulting mixture was added dropwise to a solution of lysozyme (0.1 g; 6 x $10^{-6}$ mol) dissolved in 5 ml of 0.1 N solution of sodium carbonate. The product was purified by gel filtration on a bed of Sephadex G-25 (column 14 mm x 900 mm), using 0.1 N solution of $Na_2CO_3$ as a mobile phase. The fraction A1 having an efflux time of 320-380 minutes was collected. The obtained fraction was freeze-dried to yield 40 mg of fraction A1.

Electrophoresis on an agarose gel (Lysozyme $R_f$ = -0.2; carboxyfluorescein $R_f$ = 0.6):

Fraction A1 $R_f$ = 0.1.

**Claims**

1. Inner quaternary N-triazinylammonium salts of sulphonic acids, of the Formula (1)

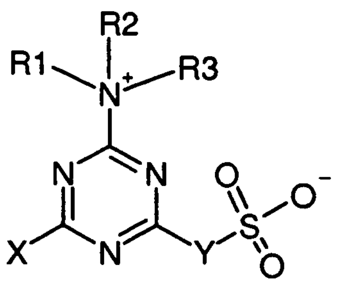

wherein
$R_1$, $R_2$ and $R_3$, each independently, represent alkyl, substituted alkyl, cycloalkyl, substituted cycloalkyl, bicycloalkyl, substituted bicycloalkyl, aryl, substituted aryl, heterocyclyl, substituted heterocyclyl, or $R_1$, $R_2$ and $R_3$ or two of them form a cyclic system or together with an oxygen, nitrogen or sulphur atom form a heterocyclic system,
X represents a substituted or unsubstituted alkoxy, cycloalkoxy, substituted or unsubstituted alkylamino, dialkylamino, alkylarylamino, arylamino or diarylamino, and
Y represents an aliphatic chain or an aromatic or heteroaromatic ring linked to a triazine ring *via* a heteroatom.

2. Salts according to claim 1, wherein the aliphatic chain in the substituent Y represents a C2-C10 chain.

3. Salts according to claim 1, wherein the ring in the substituent Y represents a 6-membered ring.

4. Salts according to claim 1, wherein X represents methoxy.

5. Salts according to claim 1, wherein $R_1$, $R_2$ and $R_3$ substituents form a mono- or bicyclic system or a mono- or heterobicyclic system.

6. Salts according to claim 1, selected from a group comprising inner salts of N-[2-(4-sulphonatephenylamino)-4-methoxy-1,3,5-triazin-6-yl]-4-methylmorpholinium, N-[2-(3-sulphonatephenylamino)-4-methoxy-1,3,5-triazin-6-yl]-4-methylmorpholinium, N-[2-(2-sulphonatephenylamino)-4-methoxy-1,3,5-triazin-6-yl]-4-methylmorpholinium, N-[2-(2-sulphonatenaphth-8-ylamino)-4-methoxy-1,3,5-triazin-6-yl]-4-methylmorpholinium, N-[2-(1-sulphonatenaphth-4-ylamino)-4-methoxy-1,3,5-triazin-6-yl]-4-methylmorpholinium and 2-[(2-sulphonate-ethylamino)-4-methoxy-1,3,5-triazin-6-yl]-4-methylmorpholinium.

7. A method of preparation of inner quaternary N-triazinylammonium salts of sulphonic acids of the Formula 1, as defined in claim 1, comprising the steps wherein:

   a) the sulphonic acid salt is treated with a chlorotriazine to form the corresponding triazine derivative,
   b) the triazine derivative obtained in step (a) is treated with a tertiary amine,
   wherein the reaction is carried out in water or in a mixture of water with tetrahydrofuran or acetonitrile, for 2 hours to 14 days, at a nearly equimolar stoichiometry of substrates, and
   wherein step (b) may optionally be preceded by a step of isolation of the triazine derivative from the reaction medium.

8. A method according to claim 7, wherein an amino derivative sulphonic acid salt is used as the sulphonic acid salt, and an aminotriazine is the triazine derivative obtained in step (a).

9. A method according to claim 7, wherein 2,4-dichloro-6-methoxy-1,3,5-triazine is used as a chlorotriazine.

10. A method according to claim 7, wherein N-methylmorpholine is used as a tertiary amine.

11. A method according to claim 7, wherein the reaction

is carried out at a temperature of 0-10°C.

**12.** A method according to claim 7, wherein the product is obtained in the form of a mixture with inorganic salts.

**13.** Use of inner quaternary N-triazinylammonium salts of sulphonic acids of the Formula 1, as defined in claim 1, as condensation reagents in the condensation reactions.

**14.** Use according to claim 13, wherein the salts are used in the reactions of peptide, ester or amide synthesis, as well as for protein modification.

**Patentansprüche**

**1.** Innere quartäre N-Triazinylammoniumsalze der Sulfonsäuren der Formel (1)

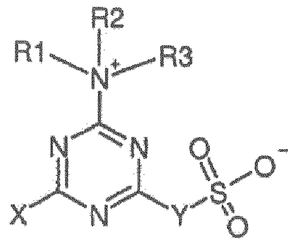

worin
$R_1$, $R_2$ und $R_3$, jeweils unabhängig voneinander, Alkyl, substituiertes Alkyl, Cycloalkyl, substituiertes Cycloalkyl, Bicycloalkyl, substituiertes Bicycloalkyl, Aryl, substituiertes Aryl, Heterocyclyl, substituiertes Heterocyklyl darstellen; oder $R_1$, $R_2$ und $R_3$ oder zwei davon ein cyclisches System bilden, oder zusammen mit einem Sauerstoff-, Stickstoff- oder Schweffelatom ein heterocyclisches System bilden;
X substituiertes oder unsubstituiertes Alkoxy, Cycloalkoxy, ein substituiertes oder unsubstituiertes Alkylamino, Dialkylamino, Alkylarylamino, Arylamino oder Diarylamino darstellt; und
Y eine aliphatische Kette oder einen aromatischen oder heteroaromatischen Ring, der an Triazinring über ein Heteroatom gebunden ist, darstellt.

**2.** Salze gemäß Anspruch 1, wobei die aliphatische Kette in dem Substituenten Y eine C2-C10-Kette darstellt.

**3.** Salze gemäß Anspruch 1, wobei der Ring in dem Substituenten Y einen 6-gliedrigen Ring darstellt.

**4.** Salze gemäß Anspruch 1, wobei X Methoxy darstellt.

**5.** Salze gemäß Anspruch 1, wobei die Substituenten $R_1$, $R_2$ und $R_3$ ein mono- oder bicyclisches System oder ein mono- oder heterobicyclisches System bilden.

**6.** Salze gemäß Anspruch 1, die aus einer Gruppe ausgewält sind, die innere Salze von N-[2-(4-Sulfonat-phenylamin)-4-methoxy-1,3,5-triazin-6-yl]-4-methylmorpholinium, N-[2-(3-Sulfonat-phenylamin)-4-methoxy-1,3,5-triazin-6-yl]-4-methylmorpholinium, N-[2-(2-Sulfonat-phenylamin)-4-methoxy-1,3,5-triazin-6-yl]-4-methylmorpholinium, N-[2-(2-Sulfonat-naphth-8-ylamin)-4-methoxy-1,3,5-triazin-6-yl]-4-methylmorpholinium, N-[2-(1-Sulfonat-naphth-4-ylamin)-4-methoxy-1,3,5-triazin-6-yl]-4-methylmorpholinium, und N-[2-(2-Sulfonat-ethylamin)-4-methoxy-1,3,5-triazin-6-yl]-4-methylmorpholinium enthält.

**7.** Verfahren zur Herstellung von inneren quartären N-Triazinylammoniumsalzen der Sulfonsäuren der Formel (1), wie in Anspruch 1 definiert, umfassend folgende Schritte, wobei:

a) das Salz der Sulfonsäure mit einem Chlorotriazin behandelt wird um das entsprechende Triazinederivat zu bilden,
b) das im Schritt (a) erhaltene Triazinderivat mit tertiärem Amin behandelt wird, wobei die Reaktion in Wasser oder in einem Gemisch von Wasser und Tetrahydrofuran oder Acetonitril, für 2 Stunden bis 14 Tage, bei einer nahezu äquimolaren Stöchiometrie der Reaktanden, durchgeführt wird, und
wobei dem Schritt (b) gegebenfalls ein Schritt der Isolierung des Triazinderivats aus dem Reaktionmedium vorausgehen kann.

**8.** Verfahren gemäß Anspruch 7, wobei als das Salz der Sulfonsäure ein Aminderivat des Salzes der Sulfonsäure verwendet wird, und das im Schritt (a) erhaltene Triazinderivat ein Aminotriazin ist.

**9.** Verfahren gemäß Anspruch 7, wobei als Chlorotriazin 2,4-Dichloro-6-methoxy-1,3,5-triazin verwendet wird.

**10.** Verfahren gemäß Anspruch 7, wobei als tertiäres Amin N-Methylmorpholin verwendet wird.

**11.** Verfahren gemäß Anspruch 7, wobei die Reaktion bei einer Temperatur von 0-10°C durchgeführt wird.

**12.** Verfahren gemäß Anspruch 7, wobei das Produkt in Form eines Gemisches mit inorganischen Salzen erhalten wird.

**13.** Verwendung der inneren quartären N-Triazinylammoniumsalze der Sulfonsäuren der Formel (1), wie

in Anspruch 1 definiert, als Kondensationsreagenzien in Kondensationsreaktionen.

**14.** Verwendung gemäß Anspruch 13, wobei die Salze bei Reaktionen der Peptid-, Ester- oder Amidsynthese, sowie zur Proteinmodifikation verwendet werden.

**Revendications**

**1.** Sels internes du N-triazinylammonium quaternaire des acides sulfoniques, de la formule (1)

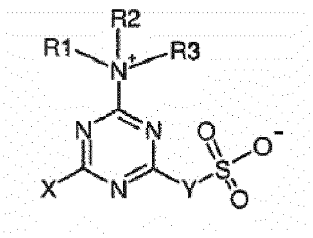

dans laquelle
$R_1$, $R_2$ et $R_3$, chacun indépendamment, représente alkyle, alkyle substitué, cycloalkyle, cycloalkyle substitué, bicycloalkyle, bicycloalkyle substitué, aryle, aryle substitué, hétérocyclyle, hétérocyclyle substitué, ou $R_1$, $R_2$ et $R_3$ ou deux d'entre eux forment un système cyclique ou ensemble avec un atome d'oxygène, d'azote ou de soufre forment un système hétérocyclique,
X représente un alcoxy substitué ou non substitué, cycloalcoxy, alkylamino substitué ou non substitué, dialkylamino, alkylarylamino, arylamino ou diarylamino, et
Y représente une chaîne aliphatique ou un cycle aromatique ou hétéro-aromatique lié à un cycle triazine par un hétéroatome.

**2.** Sels selon la revendication 1, dans lesquels la chaîne aliphatique dans le substituant Y représente une chaîne en C2-C10.

**3.** Sels selon la revendication 1, dans lesquels le cycle dans le substituant Y représente un cycle à 6 chaînons.

**4.** Sels selon la revendication 1, dans lesquels X représente méthoxy.

**5.** Sels selon la revendication 1, dans lesquels des substituants $R_1$, $R_2$ et $R_3$ forment un système mono- ou bicyclique ou un système mono- ou hétérobicyclique.

**6.** Sels selon la revendication 1, choisis parmi un groupe comprenant des sels internes de N-[2-(4-sulfonate phénylamino)-4- méthoxy-1, 3, 5-triazine-6-yl]-4-méthylmorpholinium, N-[2-(3-sulfonate phénylamino)-4-méthoxy-1, 3, 5-triazine-6-yl]-4-méthylmorpholinium, N-[2-(2-sulfonate phénylamino)-4-méthoxy-1, 3, 5-triazine-6-yl]-4-méthylmorpholinium, N-[2-(2- sulfonate napht-8-ylamino)-4-méthoxy-1, 3, 5-triazine-6-yl]-4-méthylmorpholinium, N-[2-(1-sulfonate napht-4-ylamino)-4-méthoxy-1, 3, 5-triazine-6-yle]-4-méthylmorpholinium et 2-[(2-sulfonate-éthylamino)-4-méthoxy-1, 3, 5-triazine-6-yle]-4-méthylmorpholinium.

**7.** Procédé de préparation des sels internes du N-triazinylammonium quaternaire des acides sulfoniques, de la formule 1, tels que définis dans la revendication 1, comprenant les étapes dans lesquelles:

a) le sel d'acide sulfonique est traité avec une chlorotriazine pour former le dérivé de triazine correspondant,
b) le dérivé de triazine obtenu dans l'étape (a) est traité avec une amine tertiaire,
dans lequel la réaction est réalisée dans l'eau ou dans un mélange d'eau avec du tétrahydrofuranne ou de l'acétonitrile, pendant 2 heures à 14 jours, à une stoechiométrie de substrats presque équimolaire, et
dans lequel l'étape (b) peut éventuellement être précédée d'une étape d'isolement du dérivé de triazine à partir du milieu réactionnel.

**8.** Procédé selon la revendication 7, dans lequel un sel d'acide sulfonique dérivé de l'amine est utilisé comme sel d'acide sulfonique, et une aminotriazine est le dérivé de triazine obtenu dans l'étape (a).

**9.** Procédé selon la revendication 7, dans lequel le 2,4-dichloro-6-méthoxy-1,3,5-triazine est utilisée comme une chlorotriazine.

**10.** Procédé selon la revendication 7, dans lequel N-méthylmorpholine est utilisée comme une amine tertiaire.

**11.** Procédé selon la revendication 7, dans lequel la réaction est réalisée à une température de 0-10°C.

**12.** Procédé selon la revendication 7, dans lequel le produit est obtenu sous forme d'un mélange avec des sels inorganiques.

**13.** Utilisation de sels internes N-triazinylammonium quaternaire d'acides sulfoniques de formule 1, tels que définis dans la revendication 1, en tant que réactifs de condensation dans les réactions de condensation.

**14.** Utilisation selon la revendication 13, dans lequel les

sels sont utilisés dans les réactions de synthèse de peptide, ester ou amide, ainsi que pour la modification des protéines.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- PL P366673
- EP 1586566 A
- WO 2001096282 A1
- US 6458948 B1
- JP 47034634 A
- EP 2005055793 W
- GB 1013231 A
- JP 60181377 A
- JP 60181374 A
- GB 1104134 A
- US 3178254 A
- CH 423708


### Non-patent literature cited in the description


- *Biopolymers,* vol. 55 (2), 140-165
- *Australian Journal of Chemical Society,* 2001, vol. 54, 469
- *Journal of the Organic Chemistry,* 1998, vol. 63, 4248-4255
- *Chemistry of Heterocyclic Compounds,* 2002, vol. 38, 177-182
- *Chemistry of Heterocyclic Compounds,* 1977, vol. 13, 802-805
- *Tetrahedron,* 1999, vol. 55, 13159-13170
- *Tetrahedron Letters,* 2002, vol. 43, 3323-3326
- *Journal of the American Chemical Society,* 2005, vol. 127 (48), 16912-16920
- *Journal für Praktische Chemie,* 1990, vol. 332, 579-581